# EUROPEAN PATENT SPECIFICATION

(11) **EP 0 409 928 B1**
(45) Date of publication and mention of the grant of the patent: **21.05.1997**
(21) Application number: 90901037.3
(22) Date of filing: 29.12.1989
(51) Int. Cl.: C12N 15/29, C12N 15/81, C12P 21/00, C07H 21/04, A23L 1/236

(54) **EXPRESSION OF PROTEINACEOUS SWEETENERS IN YEAST**
EXPRESSION VON PROTEINSÜSSMITTELN IN HEFE
EXPRESSION D'EDULCORANTS PROTEIQUES DANS LA LEVURE

(30) Priority: 29.12.1988 US 291456
(43) Date of publication of application: 30.01.1991
(73) Proprietor: LUCKY, LTD., Seoul 150-010 (KR)
(72) Inventor: CHO, Joong, Myung, Concord, CA 94521 (US)
(74) Representative: Perry, Robert Edward
(86) International application number: KR8900013
(87) International publication number: WO9007580

(56) References cited:
- WO-A-88/10265
- WO-A-88/10303
- US-A- 3 998 798
- Febs Letters, Vol. 29, No. 2, January 1973 (Amsterdam), H. VAN DER WEL et al. "Characterization of the Sweet-Tasting Protein from Dioscoreophyllum Cumminsii (Stapf) Diels", pages 181-184, see page 181, Introduction; page 183, tables 1,3.

## Description

### Technical Field

The field comprises recombinant methods for production of synthetic proteinacious sweeteners and compositions.

### Background

Monellin is an intensely sweet material present in the sap of "Serendipity Berries," the fruit of the West African plant, Deoscoreophyllum comminisii. The material has been purified to homogeneity and shown to be a basic protein with a molecular weight of about 1.1x10⁴ and is completely free of carbohydrate.
Monellin is the first well characterized material along several sweet or taste modifying substances found in tropical plants. It has been characterized and shown to have two subunits of about the same size held together by non-covalent bonds. The two subunits are not identical and the flavor modifying ability of monellin is dependent upon the presence of both subunits and a single mercaptan group, which if blocked abolishes the sweetness.

Because of the uncertainties and cost of extracting natural products from plant sources, an alternative route to the production of protein sweeteners is of substantial interest. Recombinant techniques offer an opportunity to synthesize proteins of varying types. However, in employing recombinant techniques, one is required to develop a strategy for producing the gene, demonstrating successful expression of the protein in a cellular host, and isolating a product which is shown to have physiological activity.

### Relevant Literature

Morris et al., J. Biol. Chem. (1973) 248 : 534-439 describe the characterization of monellin. See also Cagan, Science (1973) 181 : 32-35 ; Wlodawer and Hodgson, Proc. Natl. Acad. Sci. USA (1975) 72 ; 398-339 ; Bohak and Li, Biochimica et Biophysica Acta (1976) 427 : 153-170 ; Hudson and Bieman, Biochem. Biophys. Res. Comm. (1976) 71 : 212-220 ; Jirgenson, Biochim. Biophys. Acta (1976) 446 : 255-261 ; and Van der Wel and Loeve, FEBS Lett. (1973) 29 : 181-183 for further characterization. U.S. patent No. 3,998,798 describes the preparation of natural monellin.

Yeast leader sequences are described by Stack et al., Nucl. Acids Res. (1984) 12 : 6001-6030 and Julius et al., Cell (1984) 37 : 1075-1089. The GAL upstream activating sequence, a cis-acting DNA element, is described in Johnston and Davis, Mot. Cell. Biol. (1984) 4 : 1440-1448, while the transcription initiation region of glyceraldehyde-3-phosphate dehydrogenase is described by Holland and Holland, J. Biol. Chem. (1979) 254 : 5466-5475 ; 9839-9845 ; and Holland et al., ibid., (1983) 258 : 5291-5299.

### SUMMARY OF THE INVENTION

A DNA construct to the invention comprises, in the 5'-3' direction:
a transcriptional initiation region functional in yeast, comprising a 5' domain from the 5' domain of the transcriptional initiation region of an inducible gene functional in yeast and the 3' domain of a yeast initiation region;
an open reading frame under the transcriptional regulation of said initiation region and comprising a signal sequence, a processing signal and a structural gene encoding a protein sweetener comprising the monellin subunits, including at least one non-conservative substitution, joined by a bond or bridge; and
a transcriptional termination region.

A novel vector is capable of stable replication in yeast cells, comprising a construct as defined above. A novel method for producing the sweetener defined above comprises growing yeast cells containing the vector in a culture medium, whereby the expression product of the structural gene is processed and secreted into the medium; and isolating the protein sweetener. The sweetener has, with respect to monellin, increased sweetness or reduced pI.

### DESCRIPTION OF SPECIFIC EMBODIMENTS

Novel methods are provided for producing synthetic monellin having altered wild type sequences as a result of a bridge between the A(I) and B(II) subunits and at least one lesion involving at least one amino-acid substitution. Constructs are provided which employ a strong transcriptional initiation region which may be a naturally-occurring region or preferably selected, so as to have two domains.

The first 5' domain may be any domain which provides for enhancement and regulation, that is, domains which provide for inducible regulation, such as heat shock genes, metallothionein genes, metabolite induced genes, and the like. Of particular interest is the GAL1,10 regulatory region, particularly the upstream activating sequence, which can be induced by employing galactose as the sole carbon source, after the host cells have been grown to high density with a different carbon source. Generally, the 5' domain will be at least 50 bp, more usually at least 75 bp and may be 500 bp or lore, although usually the regulatory region will not be greater than about 1 kbp. The sequence associated with the inducible regulation of transcription will generally be within about 400 bp, and typically within about 250 bp of the second domain.

The second 3' domain (downstream of the first domain) or the promoter region without the 5' domain may be any strong yeast functional transcriptional initiation region or promoter.
Conveniently, any of the glycolytic enzyme initiation regions may be employed, such as alcohol dehydrogenase I or II, glyceraldehyde-3-phosphate dehydrogenase, glucose-6-phosphate dehydrogenase, pyruvate kinase, triose isomerase, phosphoglucose kinase, etc. Of particuar interest is the glyceraldehyde-3-phosphate dehydrogenase. Other promoter regions include the α-amylase region, invertase region, alkaline phosphatase region, etc. The 3'domain will usually be at least about 150 bp, more usually at least about 250 bp, and usually not more than about 1.5 kbp, more usually not more than about 2kbp. The 3' domain will usually be within about 200 bp of the initiation codon, more usually within about 150 bp of the initiation codon.

The two regions may be ligated together by any convenient means. Where convenient sites exist, these may be employed. In the absence of convenient restriction sites, in vitro mutagenesis or primary repair may be imployed. Alternatively, in the absence of convenient restriction sites, alternative restriction sites may be employed and the two domains joined by use of an adaptor.

The coding region will comprise in the 5'-3' direction of transcription, the initiation codon as part of a signal sequence for secretion of the subject product, a processing signal, which provides for removal of the signal sequence and processing signal to provide the mature peptide product, and the ceding region encoding the mature peptide product.

The coding sequence will usually provide for the joining of monellin subunit B(II) at the N-terminus to subunit A(I) as the C-terminus' of the protein. (In referring to the subunits, both the natural and mutated subunits are intended.) The region of joining may be varied and may involve different amino acids.

The two subunits may be joined by a short bridge, usually of not more than 10, usually of not more than 8 amino acids, or may be joined directly without intervening amino acids, or preferably the amino acids at the juncture will be modified. The amino acids at the juncture forming the bridge will provide for a polar juncture, that is, at least 50 %, usually at least 75 % of the amino acids will be polar and conveniently, at least 25 %, generally 50 % will be amino acids naturally present at the subunit terminal.
The amino acids may come from a loop of subunits I and II.

In referring to the juncture, the juncture will include as a bridge not more than 10, usually not more than 6 amino acids of the naturally occurring sequence of the subunits. For the joining of the C-terminus of subunit II with the N-terminus of subunit I, the juncture will be at Ile(46) of subunit II and Gly(6) of subunit I with the intervening amino acids, if any, as the bridge.

Where subunit II is the N-terminus, one or more of the wild-type amino acids at the juncture may be removed or substituted from either or both of the subunits, usually not more than a total of 12 amino acids, more usually not more than a total of 10 amino acids will be removed or substituted, more usually not more than a total of 6 amino acids, where the number of amino acids for each subunit will be not more than 8, usually not more than 6. Generally not more than 75% of the removed or substituted amino acids will be associated with one of the subunits.

Bridges of interest will include :

aa¹ ₓ- aa² ₓ- aa³ ₓ- aa⁴ ₓ- aa⁵ ₓ- aa⁶ ₓ- aa⁷ ₓ- aa⁸ ₓ

where only one amino acid need be present, and the individual amino acids as defined are as follows:
aa¹ is A, d, E, K, R, or Y;
aa² is Y, A, D, E, N, Q, R, T, or S;
aa³ is N, Q, S, T, D, E, R, or Y;
aa⁴ is F, W, Y, S, T, D, E, K, or R;
aa⁵ is D, E, K, R, L or T;
aa⁶ is D, E, V, I, L, K or R;
aa⁷ is G, A, V, I, L, K or R;
aa⁸ is K or R;
where x is 0 or 1, at least one x being 1.

Compositions of interest include sequences where :
aa¹ is Y or E;
aa² is D, E, Y or K;
aa³ is N, T, A or Y;
aa⁴ is R, S, K or E;
aa⁵ is E, D or T;
aa⁶ is K, D or R;
aa⁷ is G, I or L;
aa⁸ is K or R;
For sequences having the first two amino acids Y and E, there may be from 0 to 4 x's that are 0, while for chains having different amino acids as the first two amino acids there may be from 0 to 5 x's that are 0. That is, the above chains will usually be from 3 to 8, more usually 4 to 8, amino acids,

Of particular interest is removal of the naturally occurring phenylalanine, where the juncture will be Y-E-N-E-R-E-I-K. Other bridges include Y-E-N-R-E-D-I-K; Y-K-T-R-E-D-I-K; Y-E-R-E-I-K; Y-E-N-K-K; Y-E-I-K; Y-Y-A-S-D-K-L-K; Y-A-S-D-K-L; Y-A-S-D-K; Y-S-D-K; E-D-Y-K-T-R-G-R; and E-D-Y-T-R. Usually there will be at least one Y, E, D, K or R present in the chain, more usually at least one E, D, K or R. Preferred amino acids for the bridge are Y, I, S, T, D, E, K, R, N or Q, where greater than 50% of the amino acids of the bridge will be selected from this group.

In addition to any changes at the juncture, there will be at least one lesion, that is, a substitution deletion, or insertion of one of the subunits, where the result is to reduce the pI or increase sweetness. The pI or ionization constant can be reduced by substitution of a basic amino acid with an acidic or neutral amino acid, preferably an acidic amino acid, by deleting a basic amino acid, or by inserting an acidic amino acid. Usually, the number of lesions will be fewer than 8, usually fewer than 6, preferably fewer than 4, where a lesion is a single amino acid substitution, insertion or deletion.

Of particular interest is the substitution of lysine at positions 17 or 43 with an acidic amino acid, e.g. aspartic or glutamic acid, particularly glutamic, while of less interest is substitution of arginine at position 70 or 86. Desirably, the substitutions should result in a pI of less than 8 (monellin has a pI of 9.3), preferably less than 7.5, particularly in the range of about 6-7.5, more usually in the rang of about 6.5-7.5.

Any convenient termination region may be employed which is functional in yeast. The transcriptional termination region may be from the same gene as the 3' domain of the transcriptional initiation region or other gene, such as the genes indicated as useful for the transcriptional initiation region. The choice of termination region is primarily one of convenience.

The expression construct may be joined to any convenient vector for cloning. Usually, during the synthesis of the construct, various fragments, may be cloned, the fragments isolated and analyzed, joined together, recloned, and the like. The final construction may involve a shuttle vector, which provides for replication in both a bacterium, E. coli, and yeast.

A variety of vectors functional in yeast are available or can be readily prepared, based on vectors which have been disclosed in the literature. Conveniently, the vector may have a stable replication system for replication in the yeast host or an unstable replication system or no replication system, for integration into the genome of the yeast host. Vectors which are employed may include the 2 µm plasmid replication system, a combination of a centromere, e.g. CEN3 and an ARS, or the like. For integration, only a selectable marker may be employed.

The vector will also provide for a marker which provides for selection of the host containing the construct. Markers will usually employ antibiotic resistance or impart prototrophy to an auxotrophic host. Antibiotic resistance may be to kanamycin, chloramphenicol, tetracycline, ampicillin, etc. One of more markers may be present, particularly where different markers are used for the different hosts.

The yeast hosts may include strains of Saccharomyces, particularly cerevisiae, Schizo-saccharomyces, Kluyveromyces, e.g. lactis, Candida, etc. Desirably, industrial strains are employed which have been shown to be stable in fermentation.

Depending upon the nature of the host, various techniques may be employed for transforming the expression host with the expression cassette, either by itself, or as part of a vector or other construct. The introduction of the expression cassette may be as a result of transformation which includes conjugation, transformation, transfection, and transduction ; or fusion, etc. Intact host cells, protoplasts, partially regenerated protoplasts, or the like may be employed for the introduction of the exogenous DNA.

Once the host has been transformed, it may then be grown in a selective medium, so as to select for those hosts having the marker or associated expression cassette. Where antibiotic resistance is involved, the nutrient may contain a level of the antibiotic which is cytotoxic in the absence of the antibiotic resistance gene. In the case of auxotrophic complementation, the nutrient medium lacks the necessary metabolite.

Where the product is produced and retained in the cytoplasm, after sufficient time for the cells to grow, the cells may be lysed and the desired protein obtained by conventional purification procedures. These procedures include liquid-liquid extraction, HPLC, chromatography, electrophoresis, etc. The product may then be subjected to further purification, such as gel exclusion, chromatography, etc.

The resulting product may be used in a variety of ways as a sweetener. It may be used in canned products, in conjunction with various carbonated drinks, as a powder for liquid for addition to various beverages, such as coffee, tea, or the like, in cooking, chewing gum, toothpaste, mouthwash, dental hygiene products, pharmaceuticals, meat products, e.g. ham, sausage, etc., instant soups, yogurt, desserts, cereals, animal food, etc.

The subject proteinaceous sweeteners may be formulated as a liquid or powder. As a liquid, other additives may be combined, such as stabilizers, buffers, bactericides, protease inhibitors, or the like. An aqueous medium will normally be used where the sweetner will be from about 0.1 to 90 weight% of the composition. For powders, various excipients may be added which are conventional food extenders.

The following examples are provided by way of illustration and not by way of limitation.

### EXPERIMENTAL

### 1. Oligonucleotide Synthesis, Purification, and Oligomerization of Inducible Galgap Hybrid Promoter.

The oligonucleotides shown in Figure 1 were prepared based on published sequences of the GAL upstream activating sequence, a cis-acting DNA element, and yeast glyceraldehyde-3-phosphate dehydrogenase 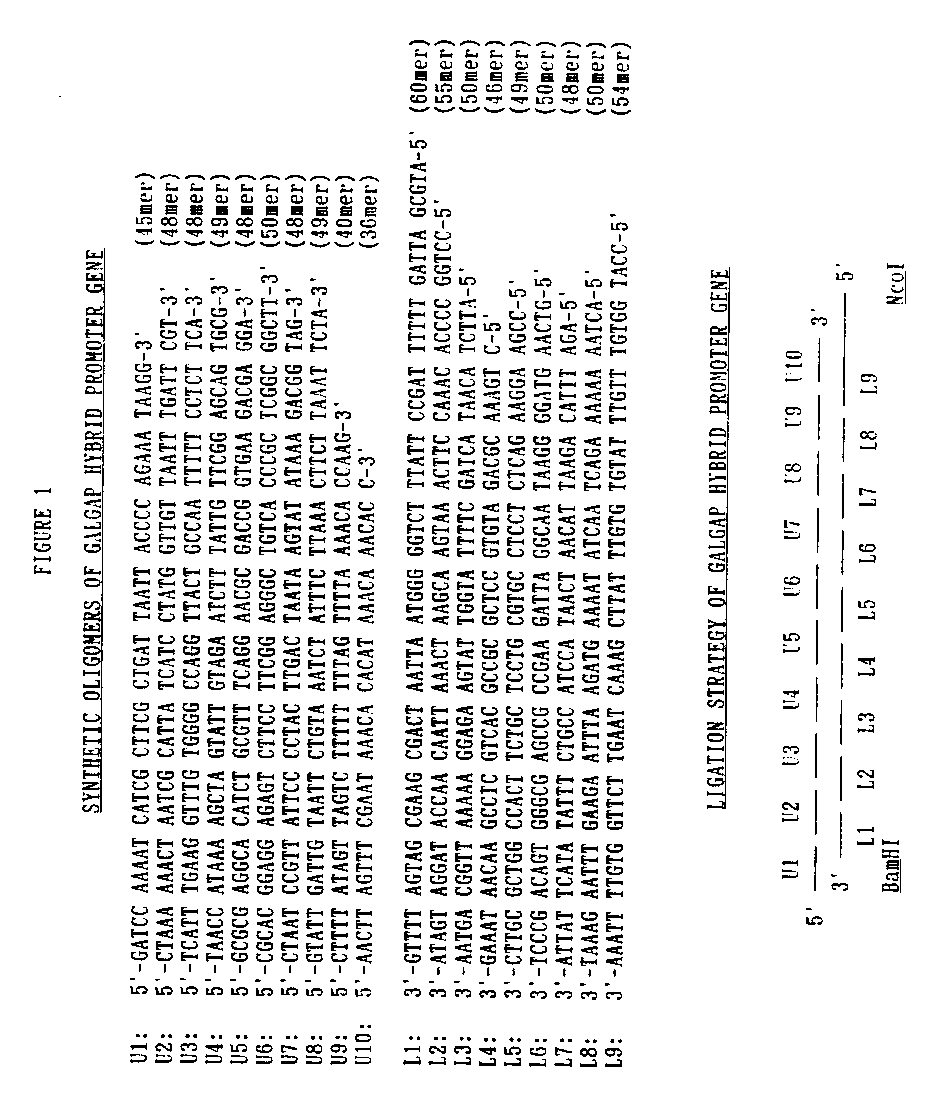
FIGURE 1

SYNTHETIC OLIGOMERS OF GALGAP HYBRID PROMOTER GENE

U1: 5'-GATCC AAAAT CATCG CTTCG CTGAT TAATT ACCCC AGAAA TAAGG-3' (45mer)
U2: 5'-CTAAA AAACT AATCG CATTA TCATC CTATG GTTGT TAATT TGATT CGT-3' (48mer)
U3: 5'-TCATT TGAAG GTTTG TGGGG CCAGG TTACT GCCAA TTTTT CCTCT TCA-3' (48mer)
U4: 5'-TAACC ATAAA AGCTA GTATT GTAGA ATCTT TATTG TTCGG AGCAG TGCG-3' (49mer)
U5: 5'-GCGCG AGGCA CATCT GCGTT TCAGG AACGC GACCG GTGAA GACGA GGA-3' (48mer)
U6: 5'-CGCAC GGAGG AGAGT CTTCC TTCGG AGGGC TGTCA CCCGC TCGGC GGCTT-3' (50mer)
U7: 5'-CTAAT CCGTT ATTCC CCTAC TTGAC TAATA AGTAT ATAAA GACGG TAG-3' (48mer)
U8: 5'-GTATT GATTG TAATT CTGTA AATCT ATTTC TTAAA CTTCT TAAAT TCTA-3' (49mer)
U9: 5'-CTTTT ATAGT TAGTC TTTTT TTTAG TTTTA AAACA CCAAG-3' (40mer)
U10: 5'-AACTT AGTTT CGAAT AAACA CACAT AAACA AACAC C-3' (36mer)

L1: 3'-GTTTT AGTAG CGAAG CGACT AATTA ATGGG GGTCT TTATT CCGAT TTTTT GATTA GCGTA-5' (60mer)
L2: 3'-ATAGT AGGAT ACCAA CAATT AAACT AAGCA AGTAA ACTTC CAAAC ACCCC GGTCC-5' (55mer)
L3: 3'-AATGA CGGTT AAAAA GGAGA AGTAT TGGTA TTTTC GATCA TAACA TCTTA-5' (50mer)
L4: 3'-GAAAT AACAA GCCTC GTCAC GCCGC GCTCC GTGTA GACGC AAAGT C-5' (46mer)
L5: 3'-CTTGC GCTGG CCACT TCTGC TCCTG CGTGC CTCCT CTCAG AAGGA AGCC-5' (49mer)
L6: 3'-TCCCG ACAGT GGGCG AGCCG CCGAA GATTA GGCAA TAAGG GGATG AACTG-5' (50mer)
L7: 3'-ATTAT TCATA TATTT CTGCC ATCCA TAACT AACAT TAAGA CATTT AGA-5' (48mer)
L8: 3'-TAAAG AATTT GAAGA ATTTA AGATG AAAAT ATCAA TCAGA AAAAA AATCA-5' (50mer)
L9: 3'-AAATT TTGTG GTTCT TGAAT CAAAG CTTAT TTGTG TGTAT TTGTT TGTGG TACC-5' (54mer)

LIGATION STRATEGY OF GALGAP HYBRID PROMOTER GENE

U1 U2 U3 U4 U5 U6 U7 U8 U9 U10
5' 3'
3' 5'
L1 L2 L3 L4 L5 L6 L7 L8 L9
BamHI NcoI
 sequences employing an Applied Biosystems DNA synthesizer model 380 B. Each oligomer was isolated from 8 M urea-polyacrylamide gel and purified with Sep-pack C18 Column (Whatman Co.).

Each oligomer was phosphorylated at 37°C for 45 min in a reaction mixture of 38 µℓ containing 50mM Tris-HCl, pH 8.0, 10 MgCI₂, 10 mM DTT, 1 mM ATP, 5 units of T4 polynucleotide kinase. The reaction mixes were pooled, extracted with an equal volume of phenol/chloroform, precipitated with 2.5 volumes of ethanol and dried under vacuum. After dissolving the dried pellet in 15 µℓ of distilled water and 7 µℓ of ligation buffer containing 0.2 mM Tris-HCI, pH 7.5, 0.1 M MgCI₂, and 0.1 mM DTT, the solution was placed in a 90°C water bath and cooled slowly to room temperature overnight. To the mixture was then added 7 µℓ of 10 mM ATP, 40 units of T4 DNA ligase and 2 µℓ of distilled water. After allowing the reaction mixture to stand at room temperature for 10 min, the DNA was extracted by phenol/chloroform, precipitated and dried as described above. The solid residue was then dissolved in 85 µℓ of distilled water and digested with BamHI and NcoI. The 462 bp fragment was isolated by 7% acrylamide gel electrophoresis, electroeluted and purified on an elutip-D column (Schleicher and Schuell Co.).

### 2. Molecular Cloning and DNA Sequencing of Galgap Hybrid Promoter.

MI3rp9 RF, a derivative of MI3mp19, which has an NcoI site between EcoRI and KpnI of MI3mp19, was used for cloning the galgap hybrid promoter gene. The gel isolated BamHI-NcoI fragment of galgap hybrid DNA was combined with NcoI-BamHI digested MI3rp9 RF in 10 µℓ of 20 mM Tris-HCI, pH 7.5, 10 mM magnesium chloride, 10 mM DTT and 200 units of T4 DNA ligase, and the ligation mixture incubated at 4°Covernignt.
Transformation of E. coli JM 101 competent cells was achieved by adding 5 µℓ of a ligation mixutre to 200 µℓ of the cells and the dideoxy DNA sequencing and KI3rp9-GG RF ligation product preparation were done as described by Messing, Methods in Enzymology (1983) 101:20-78 ; Sanler et al. Proc. Natl. Acad. Sci. USA (1985) 74:5463-5467. The promoter region had the sequence shown in Figure 2.

FIGURE 2
galgap
10 20 30 40 50
GATCAAAAAT CATCGCTTCG CTGATTAATT ACCCCAGAAA TAAGGCTAAA
60 70 80 90 100
AAACTAATCG CATTATCATC CTATGGTTGT TAATTTGATT CGTTCATTTG
110 120 130 140 150
AAGGTTTGTG GGGCCAGGTT ACTGCCAATT TTTCCTCTTC ATAACCATAA
160 170 180 190 200
AAGCTAGTAT TGTAGAATCT TTATTGTTCG GAGCAGTGCG GCGCGAGGCA
210 220 230 240 250
CATCTGCGTT TCAGGAACGC GACCGGTGAA GACGAGGACG CACGGAGGAG
260 270 280 290 300
AGTCTTCCTT CGGAGGGCTG TCACCCGCTC GGCGGCTTCT AATCCGTTAT
310 320 330 340 350
TCCCCTACTT GACTAATAAG TATATAAAGA CGGTAGGTAT TGATTGTAAT
360 370 380 390 400
TCTGTAAATC TATTTCTTAA ACTTCTTAAA TTCTACTTTT ATAGTTAGTC
410 420 430 440 450
TTTTTTTTAG TTTTAAAACA CCAAGAACTT AGTTTCGAAT AAACACACAT
460
AAACAAACAC CATGG

### Cassette Construction For Protein Sweetener Gene Expression

The synthetic galgap hybrid DNA promoter (462 bp) was isolated from M13rp9 RF-galgap and purified with a 7% polyacrylamide gel as described above. The protein sweetener gene which was synthesized, cloned and expressed in E. coli, was isolated from the ptrp-MON-1 plasmid by digestion with ClaI and SalI to obtain a partial protein sweetener gene gene fragment. (See U.S. Application Serial No. 064341, filed June 19, 1987.)

Fused Monellin
10
Gly Glu Trp Glu Ile Ile Asp Ile Gly Pro Phe Thr Gln Asn Leu
20 30
Gly Lys Phe Ala Val Asp Glu Glu Asn Lys Ile Gly Gin Tyr Gly
40
Arg Leu Thr Phe Asn Lys Val Ile Arg Pro Cys Met Lys Lys Thr
50 60
Ile Tyr Glue Asn Glu Arg Glu Ile Lys Gly Tyr Glu Tyr Gln Leu
70
Tyr Val Tyr Ala Ser Asp Lys Leu Phe Arg Ala Asp Ile Ser Glu
80 90
Asp Tyr Lys Thr Arg Gly Arg Lys Leu Leu Arg Phe Asn Gly Pro
Val Pro Pro Pro

A synthetic adapter for the NcoI/ClaI fragment of N-terminal of protein sweetner gene was synthesied with the following sequence.

5'-CATG GGT GAA TGG GAA ATT AT -3'
3'- CCA CTT ACC CTT TAA TAGC -5'
↑ ↑
NcoI ClaI

Plasmid pLBC is a derivative of pBR322 and prepared as follows. After digesting pBR322 of EcoRI and SalI, the sites were converted to BamHI sites by inserting a synthetic linker to provide a pBR322 with a single BamHI site. Plasmid pGAP, which is a derivative of pLBC in which 1300 base pairs of the BamHI fragment containing a glyceraldehyde-3-phosphate dehydrogenase (GAP) gene (Holland and Holland, supra : Holland et al., supra) promoter and terminator were inserted at the BamHI site of pLBC. The resulting vector (pGAP) comprises about 400 bp of GAP promoter, the 5' untranslated region of the GAP gene and about 900 bp of GAP terminator, the 3' untranslated region of the GAP gene. These two fragments were linked using a synthetic adapter (33 mer, 5'-CCA TGG GGT ACC CGG GGA TCC TCT AGA GTC GAC-3') for the NcoI site at the GAP initiator mehtionine at one end and the SalI site at the other end for the natural SalI site of the 3' untranslated region of the GAP gene. A SalI-BamHI fragment containing about 900 bp of GAP terminator was obtained from pGAP and isolated from a 1% agarose gel.

A ClaI-SalI fragment from ptrp322H MON-1 carrying the sweetener gene, an NcoI-ClaI adapter, (see above), a BamHI-NcoI 462 bp fragment from M13rp9-GG providing the galgap promoter region, and a SalI-BamHI partial digestion fragment (about 4.5 kbp) comprising about 900 bp of the GAP terminator region were ligated together to form an expresion cassette and cloned into pLBC to provide pGG-MON. The expression cassette excised from pGG-MON with BamHI was inserted into the BamHI site of pYLBC, an E.coli-yeast shuttle vector (pYLBC is a derivative of pJDB219 (Beggs, Nature (1978) 275:104-109) in which the region corresponding to bacterial plasmid was replaced by the entire pBR322). The plasmid pYLBC has a complete yeast 2 µm replication system and the yeast LEU2 gene. The resulting plasmid was named pYGG-MON.

### DNA Synthesis and Construction of a Secretion Cassette

A yeast leader sequence and processing signal was synthesized based on the sequence described by Stack et al., Nucleic Acids Res. (1984) 12:6011-6030 and Julius et. al., Cell(1984) 37:1075-1089. The DNA sequence as confirmed by the M13 dideoxy DNA Sequencing method is as follows:

(NcoI)
M N I F Y I F L F L L S F
5' -c ATG AAT ATA TTT TAC ATA TTT TTG TTT TTG CTA AGC TTC
3' - TTA TAT AAA ATG TAT AAA AAC AAA AAC GAT TCG AAG
Processing Site
K R
AAG AGA GGA GAA TTG GAA ATT AT - 3'
TTC TCT CCT CTT ACC CTT TAA TAG - 5'
↑
ClaI

The 63 bp fragment of leader and processing signal sequences was isolated from 8 M urea-acrylamide gel and ligated with NcoI-ClaI digested plasmid pGG-MON (see above) in the presence of 50 mM Tris-HCI, pH 8.0. 10 mM DTT, 1 mM ATP and 5 units of T4 DNA ligase to provide pGGKL-MON. The recombinant secretion vector was obtained after screening of E. coli HB101 transformants. The secretion cassette was excised by digestion of pGGKL-MON with BamHI and the fragment ligated into the BamHI site of pYLBC to provide pYGGKL-MON.

### Yeast Transformation, Growth and Secretion of Protein Sweetener

Saccharomyces cerevisiae DC04 (α MAT ade1 leu2), DBY746 (MAT α, his3-1, Leu2-3, leu2-112, ura3-52, trp1-289) were obtained from the Yeast Genetic Stock Center (University of California, Berkeley, CA). Plasmid DNA (pYGGKL-MON) was introduced into yeast cells as described by Hinnen, et al., Proc. Natl. Acad. Sci U.S.A. (1978) 75:1929-1933. The transformants were grown overnight in 3 ml culture medium containing synthetic medium deficient in leucine (Sherman, et al., Methods in Yeast Genetics, Cold Spring Harbor Laboratory, Cold Spring Harbor, NY, 1982). The overnight culture was inoculated in YEPD medium (20 g Bactopeptone, 10 g yeast extracts, 20 g glucose per liter) and grown to an O.D.650 nm of around 10. Galactose was added to provide a final concentration of 2% in the culture and the culture incubated further until an O.D. 650 nm of around 25 was obtained.

### Identification of a Protein Sweetener Internally

### Expressed or Secreted from Saccharomyces cerevisiae

To the induced cell suspension (100 µℓ) or culture supernant (100 µℓ), 50 µℓ or 3X protein SDS-sample buffer (60 mM Tris-HCI, pH 6.8, 2.3% SDS, 10% glycerol, 2% β-mercaptoethanol) was added. The mixture was boiled for 5 min and 10 µℓ loaded onto a 15 µℓ SDS-polyacrylamide gel (Laemmli, Nature (1977) 227:680-685). The gel was stained with 0.05% Coomassie brilliant blue R-250 in methanol/acetic acid/water (4:1:5, v/v), and destained in the same solution without dye. The gel showed a band of a protein of about the correct molecular weight (11 kilodalton) which band was absent in a sample from a control culture.

An effective expression system is provided for producing novel protein sweetener. The products are secreted, so as to be really isolatable and purified which provides for a protein used as a food additive for sweetening a wide variety of rpoducts. The products fold properly, so as to be obtained in active form, without requiring extensive renaturation.

## Claims

1. A DNA expression construct comprising, in the 5'-3' direction:
a transcriptional initiation region functional in yeast, comprising a first domain containing an enhancer region and a regulatory region of an inducible gene functional in yeast and downstream of the first domain, a second domain containing a yeast initiation region;
an open reading frame under the transcriptional regulation of said initiation region and comprising a signal sequence, a processing signal and a structural gene encoding a protein sweetener comprising the monellin subunits, including at least one non-conservative substitution, joined by a bond or bridge; and
a transcriptional termination region;
whereby the product expressed has increased sweetness or reduced pI, with respect to monellin.

2. A construct according to claim 1, wherein the 3' domain is the 3' domain of a yeast glycolytic enzyme.

3. A construct according to claim 2, wherein the yeast glycolytic enzyme is glyceraldehyde-3-phosphate dehydrogenase.

4. A construct according to any preceding claim, wherein the inducible 5' domain is a metabolite-inducible domain.

5. A construct according to claim 4, wherein the metabolite is D-galactose.

6. A construct according to any preceding claim, wherein the signal sequence is the partial signal sequence of Kluveromyces lactis killer toxin, and the processing signal is Lys-Arg.

7. A vector, capable of stable replication in yeast cells, comprising a construct according to any preceding claim.

8. Yeast cells comprising a construct according to any of claims 1 to 6.

9. Yeast cells according to claim 8, wherein the yeast is Saccharomyces cerevisiae.

10. A method for preparing a protein sweetener as defined in claim 1, which comprises:
growing yeast cells according to claim 8 or claim 9 in a culture medium, whereby the expression product of the structural gene is processed and secreted into the medium; and
isolating the protein sweetener.

## Patentansprüche

1. DNA-Expressionskonstrukt, das in der 5'-3'-Richtung umfaßt:
eine in Hefe funktionelle Transkriptionsinitiierungsregion umfassend eine erste Domäne, die einen Verstärkungsbereich und einen Regulationsbereich eines induzierbaren in Hefe funktionellen Gens enthält, und stromabwärts von der ersten Domäne eine zweite Domäne, die eine Hefe-Initiierungsregion enthält;
einen offenen Leserahmen unter der Transkriptionsregulation der Initiierungsregion, die eine Signalsequenz, ein Processing-Signal und ein Strukturgen umfaßt, das ein Proteinsüßmittel kodiert, das die Monellin-Untereinheiten umfaßt, einschließlich mindestens einer nicht konservativen Substitution, gebunden durch eine Bindung oder Brücke; und
eine Transkriptionsterminierungsregion;
wobei das exprimierte Produkt eine erhöhte Süßkraft oder einen verringerten pI im Hinblick auf Monellin besitzt.

2. Konstrukt nach Anspruch 1, dadurch gekennzeichnet, daß die 3'-Domäne die 3'-Domäne eines Hefe-glycolytischen Enzyms ist.

3. Ein Konstrukt nach Anspruch 2, dadurch gekennzeichnet, daß das Hefe-glycolytische Enzym Glycerinaldehyd-3-phosphatdehydrogenase ist.

4. Konstrukt nach einem der vorhergehenden Ansprüche, dadurch gekennzeichnet, daß die induzierbare 5'-Domäne eine Metabolitinduzierbare Domäne ist.

5. Konstrukt nach Anspruch 4, dadurch gekennzeichnet, daß der Metabolit D-Galactose ist.

6. Konstrukt nach einem der vorhergehenden Ansprüche, dadurch gekennzeichnet, daß die Signalsequenz die partielle Signalsequenz von Kluveromyces lactis Killertoxin ist, und das Processing-Signal Lys-Arg ist.

7. Vektor, der zu einer stabilen Replikation in Hefezellen fähig ist, umfassend ein Konstrukt nach einem der vorhergehenden Ansprüche.

8. Hefezellen, dadurch gekennzeichnet, daß sie ein Konstrukt nach einem der Ansprüche 1 bis 6 umfassen.

9. Hefezellen nach Anspruch 8, dadurch gekennzeichnet, daß die Hefe Saccharomyces cerevisiae ist.

10. Verfahren zur Herstellung eines wie im Anspruch 1 definierten Proteinsüßmittels, umfassend:
Wachsenlassen von Hefezellen gemäß Anspruch 8 oder 9 in einem Kulturmedium, wobei das Expressionsprodukt des Strukturgens einem Processing unterworfen und in das Medium sekretiert wird; und
Isolieren des Proteinsüßmittels.

## Revendications

1. Assemblage pour l'expression d'ADN comprenant, dans le sens 5'-3':
une région d'initiation de la transcription fonctionnelle dans une levure, comprenant un premier domaine comprenant une région amplificatrice et une région régulatrice d'un gène inductible fonctionnel dans une levure et, en aval du premier domaine, un second domaine contenant une région d'initiation d'une levure;
un cadre de lecture ouvert sous la régulation transcriptionnelle de ladite région d'initiation et comprenant une séquence signal, un signal de maturation et un gène de structure codant pour un édulcorant protéinique comprenant les sous-unités de la monelline, contenant au moins une substitution non conservatrice, reliées par une liaison ou un pont; et
une région de terminaison de la transcription;
le produit exprimé ayant un pouvoir édulcorant renforcé ou un pI réduit par rapport à la monelline.

2. Assemblage selon la revendication 1, dans lequel le domaine en 3' est le domaine en 3' d'une enzyme glycolytique de levure.

3. Assemblage selon la revendication 2, dans lequel l'enzyme glycolytique de levure est la glycéraldéhyde-3-phosphate déshydrogénase.

4. Assemblage selon l'une quelconque des revendications précédentes, dans lequel le domaine en 5' inductible est un domaine inductible par un métabolite.

5. Assemblage selon la revendication 4, dans lequel le métabolite est le D-galactose.

6. Assemblage selon l'une quelconque des revendications précédentes, dans lequel la séquence signal est la séquence signal partielle de la toxine tueuse de *Kluyveromyces lactis,* et le signal de maturation est Lys-Arg.

7. Vecteur, capable d'une réplication stable dans les cellules de levure, comprenant un assemblage selon l'une quelconque des revendications précédentes.

8. Cellules de levure comprenant un assemblage selon l'une quelconque des revendications 1 à 6.

9. Cellules de levure selon la revendication 8, la levure étant *Saccharomyces cerevisiae.*

10. Procédé de préparation d'un édulcorant protéinique tel que défini dans la revendication 1, qui comprend:
la culture de cellules de levure selon la revendication 8 ou la revendication 9 dans un milieu de culture, grâce à quoi le produit d'expression du gène de structure est rendu mature et sécrété dans le milieu; et
l'isolement de l'édulcorant protéinique.
